# FASCICULE DE BREVET EUROPEEN

(11) **EP 0 953 344 B1**
(45) Date de publication et mention de la délivrance du brevet: **26.05.2004**
(21) Numéro de dépôt: 99400860.5
(22) Date de dépôt: 08.04.1999
(51) Int. Cl.: A61K 7/48, A61K 31/05

(54) **Utilisation d'au moins un hydroxystilbène dans une composition destinée à favoriser la desquamation de la peau.**
Verwendung mindestens eines Hydroxystilben in einer Zusammensetzung, die die Abschuppung der Haut fördern soll.
Use of at least a hydroxystilbene in a composition to stimulate desquamation of the skin.

(30) Priorité: 10.04.1998 FR 9804569
(43) Date de publication de la demande: 03.11.1999
(73) Titulaire: L'OREAL, 75008 Paris (FR)
(72) Inventeur: Breton, Lionel, 78000 Versailles (FR); Pineau, Nathalie, Rés. des Jardins du Clain, 86000 Poitiers (FR)
(74) Mandataire: Tezier Herman, Béatrice

(56) Documents cités:
- PATENT ABSTRACTS OF JAPAN vol. 096, no. 011, 29 novembre 1996 (1996-11-29) & JP 08 175960 A (KAO CORP), 9 juillet 1996 (1996-07-09)
- BOMBARDELLI ET AL.: "Vitis vinifera L" FITOTERAPIA, vol. 66, no. 4, 1995, pages 291-317, XP002095075 italia
- DATABASE WPI Week 1098 Derwent Publications Ltd., London, GB; AN 98-105073 XP002095188 & JP 09 328410 A (MITSUBA BOEKI KK.), 22 décembre 1997 (1997-12-22)
- PATENT ABSTRACTS OF JAPAN vol. 015, no. 297 (C-0854), 29 juillet 1991 (1991-07-29) & JP 03 109343 A (MARUZEN KASEI CO LTD), 9 mai 1991 (1991-05-09)

## Description

L'invention concerne l'utilisation en tant que composé actif dans une composition ou pour la préparation d'une composition, d'une quantité efficace d'au moins un hydroxystilbène, cet hydroxystilbène ou la composition étant destinés à favoriser la desquamation de la peau. Elle se rapporte aussi à des compositions pour favoriser la desquamation de la peau et/ou stimuler le renouvellement épidermique, ainsi qu'à un procédé de traitement non thérapeutique de la peau destiné à favoriser la desquamation et/ou lutter contre le vieillissement de la peau.

La desquamation est un phénomène naturel lié au fait que l'épiderme, qui constitue la couche supérieure de la peau, est en constante régénération. L'épiderme est constitué de plusieurs assises de cellules, dont la plus profonde est l'assise basale constituée de cellules indifférenciées. Au cours du temps, ces cellules vont différencier et migrer vers la surface de l'épiderme en constituant les différentes assises de celui-ci, jusqu'à former à la surface de l'épiderme les coméocytes, cellules mortes qui constituent la dernière couche de l'épiderme, c'est à dire la couche la plus externe encore appelée *stratum corneum* qui s'éliminent par desquamation. Cette perte en surface est compensée par la migration de cellules de l'assise basale vers la surface de l'épiderme. Il s'agit du renouvellement perpétuel de la peau.
Une peau jeune renouvelle ses couches superficielles toutes les deux à trois semaines, alors qu'une peau mature peut prendre deux fois plus de temps. Une des conséquences de ce ralentissement du renouvellement épidermique est l'impression de peau sèche. En effet, plus le processus de renouvellement est long, plus l'hydratation naturelle à la surface de la peau diminue.
De plus, plus la desquamation est ralentie, plus la peau prend un aspect mat et perd de son éclat et de sa fraîcheur.

Une élimination forcée de la couche cornée accélère le renouvellement épidermique, permet de lutter contre le vieillissement cutané et la peau retrouve alors un aspect jeune et frais ainsi qu'une meilleure hydratation. La peau perd ainsi son aspect mat et le teint s'en trouve amélioré.

Le vieillissement cutané, résultant d'effets sur la peau de facteurs intrinsèques ou extrinsèques, se traduit par l'apparition de rides et ridules, par le jaunissement de la peau, qui développe notamment un aspect parcheminé, accompagné éventuellement de l'apparition de taches pigmentaires, par la désorganisation des fibres d'élastine et de collagène entraînant une perte d'élasticité, de souplesse et de fermeté et par l'apparition de télangiectasies.

Certains de ces signes du vieillissement sont plus particulièrement liés au vieillissement intrinsèque ou physiologique, c'est-à-dire au vieillissement « normal » lié à l'âge ou chronobiologique, alors que d'autres sont plus spécifiques du vieillissement extrinsèque, c'est-à-dire du vieillissement provoqué d'une manière générale par l'environnement ; il s'agit plus particulièrement du photovieillissement dû à l'exposition au soleil, à la lumière ou à tout autre rayonnement.

Les changements de la peau dus au vieillissement intrinsèque sont la conséquence d'une sénescence génétiquement programmée où interviennent des facteurs endogènes. Ce vieillissement intrinsèque provoque notamment un ralentissement du renouvellement des cellules de la peau, ce qui se traduit essentiellement par l'apparition d'altérations cliniques telles que la réduction du tissu adipeux sous-cutané et l'apparition de fines rides ou ridules, et par des changements histopathologiques tels qu'une augmentation du nombre et de l'épaisseur des fibres élastiques, une perte de fibres verticales de la membrane du tissu élastique, et la présence de grands fibroblastes irréguliers dans les cellules de ce tissu élastique.

Au contraire, le vieillissement extrinsèque entraîne des altérations cliniques telles que des rides épaisses et la formation d'une peau molle et/ou tannée, et des changements histopathologiques tels qu'une excessive accumulation de matière élastique dans le derme supérieur et une dégénérescence des fibres de collagène.

On connaît dans l'art antérieur divers agents destinés à lutter contre le vieillissement cutané.

Ainsi, le brevet US-A-4603146 décrit l'emploi d'acide rétinoïque et de ses dérivés dans des compositions cosmétiques, en vue de lutter contre le vieillissement cutané.

Par ailleurs, de nombreux brevets et publications (voir par exemple la demande EP-A-413528) ainsi que de nombreuses compositions cosmétiques du commerce décrivent l'emploi des α-hydroxy-acides comme l'acide lactique, l'acide glycolique ou encore l'acide citrique pour traiter le vieillissement cutané.

JP08175960 décrit des compositions cosmétiques contenant des dérivés de stilbène, destinées à prévenir la formation des rides. Une association avec un agent anti-inflammatoire ou un agent antioxydant est préferée.

Le document XP-002095188 décrit une composition cosmétique comprenant un extrait de Yucca contenant des saponines, des flavones et du resvératrol. Cette composition est utile dans la prévention d'une peau rugueuse et âgée. Cette composition révèle une activité antimicrobienne et une capacité à absorber les UV.

On connaît enfin les bêta-hydroxy-acides et plus spécialement l'acide salicylique ainsi que ses dérivés pour leur propriétés desquamantes (voir les documents WO-A-93/10756 et US-A-4 767 750).

Tous ces composés ont une action contre le vieillissement de la peau en favorisant la desquamation, c'est-à-dire l'élimination des cellules « mortes » situées à la surface de la couche cornée de l'épiderme. Cette propriété "desquamante" est aussi appelée, souvent à tort, propriété kératolytique.

Mais les composés de l'art antérieur présentent également des effets secondaires, tels que des picotements, des tiraillements, des échauffements et/ou des rougeurs désagréables pour l'utilisateur.

On constate donc que subsiste le besoin d'agents antivieillissement ayant une action au moins aussi efficace que celle des composés de l'art antérieur, mais ne présentant pas leurs inconvénients.

L'invention a pour but de pallier les inconvénients des solutions de l'art antérieur et de proposer l'utilisation d'une composition favorisant la desquamation de la peau et/ou stimulant le renouvellement épidermique, dont l'utilisation n'entraînerait pas de picotements, de tiraillements, d'échauffements ou de rougeurs désagréables pour l'utilisateur.

La demanderesse a trouvé de manière inattendue que l'application d'une quantité efficace d'au moins un hydroxystilbène permet de favoriser la desquamation de la peau et/ou stimuler le renouvellement épidermique.

Les hydroxystilbènes sont des composés répondant à la formule générale I : 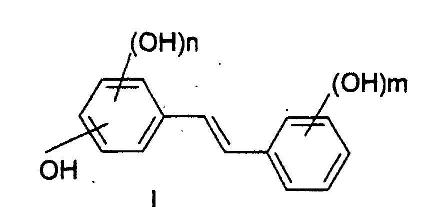 dans laquelle n est un nombre entier compris entre 0 et 4 inclusivement et m est un nombre entier compris entre 0 et 5 inclusivement. Ces composés peuvent être sous une forme Cis ou Trans.
Selon l'invention, le terme hydroxystilbène recouvre aussi bien les composés de formule I que leurs dérivés hydroxyalkylés.

Les hydroxystilbènes sont des composés que l'on retrouve à l'état naturel dans les végétaux de la classe des spermatophytes et particulièrement dans la vigne et le raisin et dans le vin.

Dans l'art antérieur les hydroxystilbènes sont utilisés entre autres comme agents dépigmentants (JP87-192040), comme agents vasodilatateurs (EP 96-830517), comme agents antithrombiques (JP 05016413), dans le traitement de diverses affections cardio-vasculaires (CA 2187990), comme agents inhibiteurs de mutagenèse (JP 06024967), ou encore décrits comme antioxydant.

Parmi ces composés, le resvératrol ou (3,4',5-trihydroxystilbène) est particulièrement étudié pour les activités ci-dessus décrites principalement parce qu'il s'agit d'un composé naturel qui se retrouve dans la peau de raisin et dans le vin. A cet égard on peut consulter la revue de Soleas et collaborateurs (Clinical Biochemistry, vol. 30, N°2, pp. 91-113, 1997) qui résume parfaitement l'état des connaissances concernant ce composé et les hydroxystilbènes.

Mais, la capacité des hydroxystilbènes à favoriser la desquamation de la peau et/ou stimuler le renouvellement épidermique n'a jamais été décrite à ce jour.

L'invention a donc pour premier objet l'utilisation en tant que composé actif dans une composition ou pour la préparation d'une composition dermatologique d'une quantité efficace d'au moins un hydroxystilbène, cet hydroxystilbène ou la composition étant destinés à favoriser la desquamation de la peau et/ou stimuler le renouvellement épidermique.

La composition est de préférence destinée à un usage cosmétique ou dermatologique, avantageusement cosmétique.

Parmi les hydroxystilbènes, on peut citer les mono, di, tri, tétra, penta, hexa, hepta, octo, nonahydroxystilbènes, ou encore leurs dérivés hydroxyalkylés.

Par principe actif, on entend toute molécule ou composition susceptible de modifier ou de moduler le fonctionnement d'au moins un système biologique donné.

Un autre objet de L'invention est un procédé de traitement non thérapeutique de la peau destiné à favoriser la desquamation de la peau et/ou stimuler le renouvellement épidermique, par application sur la peau d'au moins un hydroxystilbène ou d'une composition cosmétique en contenant.

Selon l'invention les hydroxystilbènes peuvent être utilisés seuls ou en mélanges de toute nature et peuvent être d'origine naturelle ou synthétique.

Les hydroxystilbènes utilisables selon l'invention sont choisis parmi :
le 4'-hydroxystilbène,
le 2',4'-dihydroxystilbène,
le 3',4'-dihydroxystilbène,
le 4,4'-dihydroxystilbène,
le 2',4',4-trihydroxystilbène,
le 3',4',4-trihydroxystilbène,
le 2,4,4'-trihydroxystilbène,
le 3,4,4'-trihydroxystilbène,
le 3,4',5-trihydroxystilbène,
le 2',3,4-trihydroxystilbène,
le 2,3',4-trihydroxystilbène,
le 2',2,4'-trihydroxystilbène,
le 2,4,4',5-tétrahydroxystilbène,
le 2',3,4',5-tétrahydroxystilbène,
le 2,2',4,4'-tétrahydroxystilbène,
le 3,3',4',5-tétrahydroxystilbène,
le 2,3',4,4'-tétrahydroxystilbène,
le 3,3',4,4'-tétrahydroxystilbène,
le 3,3',4',5,5'-pentahydroxystilbène,
le 2,2',4,4',6-pentahydroxystilbène,
le 2, 3',4,4',6-pentahydroxystilbène,
le 2,2',4,4',6,6'-hexahydroxystilbène.

Préférentiellement, on utilise selon l'invention du 3,4',5-trihydroxystilbène (ou encore appelé resvératrol).

La quantité d'hydroxystilbène utilisable selon l'invention dépend bien évidemment de l'effet recherché et doit être en une quantité efficace pour favoriser la desquamation de la peau et/ou stimuler le renouvellement épidermique.

A titre d'exemple la quantité d'hydroxystilbène utilisable selon l'invention peut aller par exemple de 0,001% à 10% et de préférence de 0,005% à 5% du poids total de la composition.

Selon un autre aspect, l'invention à pour objet l'utilisation d'une composition pour favoriser la desquamation de la peau et/ou stimuler le renouvellement épidermique, comprenant au moins un hydroxystilbène tel que défini précédemment.

Les hydroxystilbènes selon l'invention peuvent donc être utilisés en tant qu'agent destiné à favoriser la desquamation de la peau et/ou stimuler le renouvellement épidermique, en particulier dans une composition à usage capillaire ou dans une composition pour la peau du corps et/ou du visage.

Ladite composition peut se présenter sous la forme d'une émulsion, notamment huile-dans-eau ou eau-dans-huile, voire sous la forme d'une émulsion multiple.

Elle peut également se présenter sous forme d'une solution aqueuse, éventuellement gélifiée, ou sous forme d'une lotion, par exemple biphasée, d'une crème, d'un lait, voire d'une mousse.

La composition utilisée selon l'invention peut comprendre une phase huileuse à base d'huile animale, végétale, minérale, siliconée, fluorée et/ou synthétique.
La phase huileuse peut également comprendre des alcools gras ou des acides gras, ainsi que des tensioactifs.
On peut notamment citer les huiles hydrocarbonées telles que l'huile de paraffine ou de vaseline; le perhydrosqualène; l'huile d'arara, l'huile d'amande douce, de calophyllum, de palme, de ricin, d'avocat, de jojoba, d'olive ou de germes de céréales; des alcools tels que l'alcool oléique, l'alcool linoléique ou linolénique, l'alcool isostéarique ou l'octyl dodécanol. On peut également citer les huiles siliconées telles que les PDMS, éventuellement phénylées telles que les phényltriméthicones.
La phase huileuse peut également comprendre une huile démaquillante telle qu'un ester d'acides gras, en particulier les esters obtenus à partir d'un alcool à chaîne droite ou ramifiée ayant de 1 à 17 atomes de carbone et d'un acide gras à chaîne droite ou ramifiée ayant de 3 à 18 atomes de carbone.
Un tel ester peut en particulier être choisi dans le groupe constitué par l'adipate de dioctyle, le palmitate d'éthyl-2 hexyle, l'adipate de diisopropyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle.
La phase huileuse peut être présente à raison de 5-95% en poids dans le cas d'une émulsion.

La composition selon l'invention peut comprendre, en outre,
. un agent permettant la mise en suspension de la phase grasse, par exemple un copolymère d'acrylates d'alkyle en C₁₀-C₃₀ et d'acide acrylique ou méthacrylique ou de leur ester (Pemulen TR1, Pemulen TR2, Carbopol 1342 de GOODRICH); ou un copolymère acrylamide/acide méthylpropane sulfonique (Sepigel de SEPPIC), et/ou
. un agent de mise en dispersion de la phase grasse, tel qu'un système émulsionnant ou vésiculaire à base de vésicules, éventuellement de taille nanométrique, constituées de lipides ioniques (liposomes) où non ioniques, et en particulier les systèmes émulsionnants bien connus de l'homme du métier constitués de stéarate de glycéryl /PEG 100 stéarate (CTFA), d'alcool cétylique et d'alcool stéarylique.

La composition utilisée de l'invention peut comprendre, en outre, un agent pour modifier sa viscosité, et obtenir des textures plus ou moins gélifiées, tel que :
. les dérivés cellulosiques (carboxyméthylcellulose, hydroxyéthylcellulose, hydroxypropylméthylcellulose),
. les gommes naturelles telles que les gommes de xanthane, de guar, de caroube, les scléroglucanes, les dérivés de chitine ou de chitosane, les carraghénanes,
. les dérivés polycarboxyvinyliques du type Carbomer (vendues par GOODRICH sous les dénominations Carbopol, 940, 951, 980, ou par 3V-SIGMA sous la dénomination Synthalen K ou Synthalen L).

La composition utilisée selon l'invention peut également comprendre, de façon connue, des adjuvants couramment utilisés dans le domaine considéré, tels que les conservateurs, les antioxydants, les parfums, les charges telles que le kaolin ou l'amidon, voire les microsphères creuses, les pigments, les filtres UV, les séquestrants, les huiles essentielles, les matières colorantes, les actifs hydrophiles ou lipophiles tels que les hydratants, notamment la glycérine, le butylène glycol, les anti-inflammatoires comme l'allantoïne, le bisabolol, les anti-radicaux libres comme la vitamine E ou ses dérivés, les apaisants tels que l'eau de bleuet, l'extrait d'iris, les dépigmentants, les actifs biologiques tels que l'urée, les acides aminés, les vitamines et leurs dérivés, les protéines, l'acide salicylique et ses dérivés, les α-hydroxy-acides, l'acide pyrrolidone carboxylique et ses sels, les céramides.
Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition présente de préférence un pH qui respecte la peau, généralement compris entre 5 et 8, de préférence un pH de 5,5 à 7,5.

L'invention est illustrée plus en détails dans les exemples suivants.

### Exemple 1: On étudie, dans cet exemple, la capacité du resvératrol à favoriser la desquamation.

Ce test de l'effet *in vitro* d'un agent actif sur la desquamation est réalisé sur kératinocytes humains différenciés. Le principe du test repose sur le fait que la desquamation induit la libération de coméocytes. Le pouvoir de desquamation du produit testé va être d'autant plus grand que le nombre de coméocytes libéré sera important.

Le protocole du test a été le suivant: à partir de biopsies de peau humaine, les kératinocytes obtenus par séparation de l'épiderme sont dissociés par action enzymatique à la trypsine et mis en culture à la concentration de 2.10⁵ cellules/ml. La croissance et la différenciation des kératinocytes a été obtenue par culture durant 10 à 20 jours en milieu spécifique. Puis, après élimination du milieu de culture, l'activité du produit à tester est évalué. Pour ce faire, deux prélèvements à T0 et T60 ont été réalisés, c'est-à-dire avant l'ajout du produit et 60 minutes après cet ajout. Les prélèvements ainsi effectués ont été analysés au cytomètre de flux pour dénombrer la population de cornéocytes. Le cytomètre de flux permet de distinguer les populations de coméocytes et de kératinocytes par traitement à l'acridine orange spécifique de l'ADN des cellules. Cette coloration est spécifique des kératinocytes puisque les coméocytes normaux ne possèdent pas de noyaux donc par d'ADN.

L'indice de détachement cellulaire est déterminé par la différence entre T60 et T0. La même mesure a été réalisée pour un témoin ne contenant pas de produit à tester car l'expérience produit inévitablement la libération de cornéocytes, même en absence d'actifs.

Les résultats de ces études sont résumés dans le tableau suivant :

| | Témoin | référence* 5.10⁻⁵ M | resvératrol 5.10⁻⁵ M |
|---|---|---|---|
| Activité en % | 100 | 196,6 | 188 |
| | | p<0,05 | p<0,05 |

| | | | |
|---|---|---|---|
| * Référence : Acide 2-hydroxy-5-octanoylbenzoïque connu comme favorisant la desquamation (Brevet FR-85-06953 de la demanderesse) Témoin : tampon PBS seul. p : analyse de variance - test de comparaison multiple de Dunnett. | | | |

Les résultats sont donnés en % d'activité par rapport au témoin constitué d'une culture identique en l'absence de composé.
L'activité du resvératrol sur le détachement cellulaire est donc importante. Cette activité dose dépendante est proche (en équivalent %) de celle de la référence.

### Exemple 2 : Exemples de formulations illustrant l'invention. Ces compositions ont été obtenues par simple mélange des différents composants.

### Composition 1 : Lait pour le visage

- Huile de vaseline 7,0 g
- Resvératrol 0,1 g
- Monostéarate de glycéryle, stéarate de polyéthylène glycol (100 OE) 3,0 g
- Polymère carboxyvinylique 0,4 g
- Alcool stéarylique 0,7 g
- Protéines de soja 3,0 g
- NaOH 0,4 g
- Conservateur qs
- Eau qsp 100 g

Cette composition se présente sous forme d'un lait pour le visage, ayant de bonnes propriétés cosmétiques, et étant doux et confortable à utiliser.
Le pH de la composition est d'environ 5,5.

### Composition 2 : Lotion

- Resvératrol : 0,5 g
- Palmitate d'éthyl-2 hexyle 10,0 g
- Cyclopentadiméthylsiloxane 20,0 g
- Butylène glycol 5,0 g
- Conservateur qs
- Eau qsp 100 g

Cette lotion, qui ne contient pas de tensioactif, favorise la desquamation de la peau.

### Composition 3 : Lait

- Palmitate d'octyle 35,0 g
- Glycérine 2,0 g
- Resvératrol 0,8 g
- Polymère réticulé acrylates C10-C30/alkylacrylates 0,1 g
- Triéthanolamine 0,1 g
- Acides aminés de blé 1,0 g
- Conservateur qs
- Eau qsp 100 g

Le lait obtenu, qui ne contient pas de tensioactif, possède de bonnes propriétés cosmétiques.

### Composition 4 : Gel pour le visage

- Glycérine 10,0 g,
- Resvératrol 1,0 g
- Cocoamphodiacétate de disodium 1,0 g
- Conservateur qs
- Eau qsp 100 g

Le gel obtenu possède de bonnes propriétés cosmétiques.

### Composition 5 : Gel nettoyant à l'eau

- Butylène glycol 7,0 g
- Sarcosinate de lauroyl sodium 4,0 g
- Resvératrol 5,0 g
- Triéthanolamine 0,8 g
- Carbomer 0,5 g
- Conservateur qs
- Eau qsp 100 g

Le gel obtenu possède de bonnes propriétés cosmétiques.

## Revendications

1. Utilisation en tant que composé actif dans une composition cosmétique ou pour la préparation d'une composition dermatologique, d'une quantité efficace d'au moins un hydroxystilbène, cet hydroxystilbène ou la composition étant destinés à favoriser la desquamation de la peau et/ou stimuler le renouvellement épidermique.

2. Utilisation selon la revendication 1, **caractérisée en ce que** l'hydroxystilbène est choisi parmi :
le 4'-hydroxystilbène,
le 2',4'-dihydroxystilbène,
le 3',4'-dihydroxystilbène,
le 4,4'-dihydroxystilbène,
le 2',4',4-trihydroxystilbène,
le 3',4',4-trihydroxystilbène,
le 2,4,4-trihydroxystilbène,
le 3,4,4'-trihydroxystilbène,
le 3,4',5-trihydroxystilbène,
le 2',3,4-trihydroxystilbène,
le 2,3',4-trihydroxystilbène,
le 2',2,4'-trihydroxystilbène,
le 2,4,4',5-tétrahydroxystilbène,
le 2',3.4',5-tétrahydroxystilbène,
le 2,2',4,4'-tétrahydroxystilbène,
le 3,3',4',5-tétrahydroxystilbène,
le 2,3',4,4'-tétrahydroxystilbène,
le 3,3',4,4'-tétrahydroxystilbène,
le 3,3', 4',5, 5'-pentahydroxystilbène,
le 2,2',4,4',6-oentahydroxystilbène,
le 2, 3',4,4',6-pentahydroxystilbène,
le 2,2',4,4',6,6'-hexahydroxystilbène.

3. Utilisation selon l'une quelconque des revendications précédentes, **caractérisée en ce que** l'hydroxystilbène est du 3,4',5-trihydroxystilbène.

4. Utilisation selon l'une quelconque des revendications précédentes, **caractérisée par le fait que** l'hydroxystilbène est présent en une quantité allant de 0,001% à 10% du poids total de la composition.

5. Utilisation selon la revendication précédente, **caractérisée par le fait que** l'hydroxystilbène est présent en une quantité allant de 0,005% à 5% du poids total de la composition.

6. Utilisation selon l'une quelconque des revendications précédentes, dans une composition se présentant sous la forme d'une émulsion, d'une solution aqueuse, éventuellement gélifiée, d'une lotion notamment biphasée, d'une crème, d'un lait, d'une mousse.

7. Procédé non thérapeutique pour favoriser la desquamation de la peau et/ou stimuler le renouvellement épidermique, **caractérisé en ce qu'**on applique sur la peau une composition cosmétique comprenant une quantité efficace d'au moins un hydroxystilbène tel que défini dans l'une des revendications 1 à 5.

## Claims

1. Use, as active compound in a cosmetic composition or for the preparation of a dermatological composition, of an effective quantity of at least one hydroxystilbene, this hydroxystilbene or the composition being intended to promote desquamation of the skin and/or stimulate epidermal renewal.

2. Use according to Claim 1, **characterized in that** the hydroxystilbene is chosen from:
4'-hydroxystilbene,
2',4'-dihydroxystilbene,
3',4'-dihydroxystilbene,
4,4'-dihydroxystilbene,
2',4',4-trihydroxystilbene,
3',4',4-trihydroxystilbene,
2,4,4'-trihydroxystilbene,
3,4,4'-trihydroxystilbene,
3,4',5-trihydroxystilbene,
2',3,4-trihydroxystilbene,
2,3',4-trihydroxystilbene,
2',2,4'-trihydroxystilbene,
2,4,4',5-tetrahydroxystilbene,
2',3,4',5-tetrahydroxystilbene,
2,2',4,4'-tetrahydroxystilbene,
3,3',4',5-tetrahydroxystilbene,
2,3',4,4'-tetrahydroxystilbene,
3,3',4,4'-tetrahydroxystilbene,
3,3',4',5,5'-pentahydroxystilbene,
2,2',4,4',6-pentahydroxystilbene,
2,3',4,4',6-pentahydroxystilbene,
2,2',4,4',6,6'-hexahydroxystilbene.

3. Use according to any one of the preceding claims, **characterized in that** the hydroxystilbene is 3,4',5-trihydroxystilbene.

4. Use according to any one of the preceding claims, **characterized in that** the hydroxystilbene is present in a quantity ranging from 0.001% to 10% of the total weight of the composition.

5. Use according to the preceding claim, **characterized in that** the hydroxystilbene is present in a quantity ranging from 0.005% to 5% of the total weight of the composition.

6. Use according to any one of the preceding claims, in a composition provided in the form of an emulsion, an aqueous solution, optionally gelled, a lotion, especially a two-phase lotion, a cream, a milk or a foam.

7. Nontherapeutic method for promoting desquamation of the skin and/or stimulating epidermal renewal, **characterized in that** a cosmetic composition comprising an effective quantity of at least one hydroxylstilbene as defined in any of claims 1 to 5 is applied to the skin.

## Patentansprüche

1. Verwendung einer wirksamen Menge mindestens eines Hydroxystilben als Wirkstoff in einer kosmetischen Zusammensetzung oder zur Herstellung einer dermatologischen Zusammensetzung, wobei das Hydroxystilben oder die Zusammensetzung zur Förderung der Abschuppung der Haut und/ oder zur Stimulierung der Epidermiserneuerung vorgesehen sind.

2. Verwendung nach Anspruch 1, **dadurch gekennzeichnet, dass** das Hydroxystilben ausgewählt ist unter:
4'-Hydroxystilben,
2',4'-Dihydroxystilben,
3',4'-Dihydroxystilben,
4,4'-Dihydroxystilben,
2',4',4-Trihydroxystilben,
3',4',4-Trihydroxystilbcn,
2,4,4'-Trihydroxystilben,
3,4,4'-Trihydroxystilben,
3,4',5-Trihydroxystilben,
2',3,4-Trihydroxystilben,
2, 3',4-Trihydroxystilben,
2',2,4'-Trihydroxystilben,
2,4,4',5-Tetrahydroxystilben,
2',3,4',5-Tetrahydroxystilben,
2,2',4,4'-Tetrahydroxystilben,
3,3',4',5-Tetrahydroxystilben,
2,3',4,4'-Tetrahydroxystilben,
3,3',4,4'-Tetrahydroxystilben,
3,3',4',5,5'-Pentahydroxystilben,
2,2',4,4',6-Pentahydroxystilben,
2,3',4,4',6-Pentahydroxystilben,
2,2',4,4',6,6'-Hexahydroxystilben.

3. Verwendung nach einem der vorhergehenden Ansprüche, **dadurch gekennzeichnet, dass** es sich bei dem Hydroxystilben um das 3,4',5-Trihydroxystilben handelt.

4. Verwendung nach einem der vorhergehenden Ansprüche, **dadurch gekennzeichnet, dass** das Hydroxystilben in einer Menge von 0,001 bis 10 % des Gesamtgewichts der Zusammensetzung verwendet wird.

5. Verwendung nach dem vorhergehenden Anspruch, **dadurch gekennzeichnet, dass** das Hydroxystilben in einer Menge von 0,005 bis 5 % des Gesamtgewichts der Zusammensetzung verwendet wird.

6. Verwendung nach einem der vorhergehenden Ansprüche in einer Zusammensetzung, die in Form einer Emulsion, einer wässrigen Lösung, die gegebenenfalls in ein Gel übergeführt ist, einer Lotion, insbesondere als zweiphasige Lotion, einer Creme, einer Milch oder eines Schaums vorliegt.

7. Nicht therapeutisches Verfahren zur Förderung der Abschuppung der Haut und/oder Stimulierung der Epidermisemeuerung, **dadurch gekennzeichnet, dass** auf die Haut eine kosmetische Zusammensetzung aufgetragen wird, die mindestens ein Hydroxystilben nach einem der Ansprüche 1 bis 5 in einer wirksamen Menge enthält.
